# EUROPEAN PATENT SPECIFICATION

(11) **EP 2 191 864 B1**
(45) Date of publication and mention of the grant of the patent: **01.01.2014**
(21) Application number: 08020520.6
(22) Date of filing: 26.11.2008
(51) Int. Cl.: A61K 8/41, A61Q 5/10, A61Q 5/04, A61Q 5/06

(54) **Process for colouring and straightening keratin fibres**
Verfahren zur Färbung und dauerhaften Glättung von Keratinfasern
Procédé pour colorer et lisser des fibres de kératine

(43) Date of publication of application: 02.06.2010
(73) Proprietor: Kao Germany GmbH, 64297 Darmstadt (DE)
(72) Inventor: Nöcker, Bernd Dr., Tokyo 105-0011 (JP); Wood, Jonathan Dr., 69469 Weinheim (DE); Hullmann, Alexandra Dr., 64331 Weiterstadt (DE); Kubatz, Horst, 64665 Alsbach-Hähnlein (DE); Weißbach, Kornelia, 65812 Bad Soden (DE)
(74) Representative: Grit, Mustafa

(56) References cited:
- EP-A- 0 083 095
- EP-A- 0 226 860
- EP-A- 0 260 716
- EP-A- 1 655 056
- US-A1- 2005 076 459

## Description

The present invention is related to a process for colouring and straightening keratin fibres especially human hair in a single process.

Hair colouring is a common practice for ages. Oxidative colouration has widely been used for achieving durable, brilliant hair colour. Direct dyes, mainly of cationic character, have also found their applications for colouring hair. Recently, anionic direct dyes have as well been found to be very powerful for changing hair colour permanently and to achieve long lasting, brilliant colours in strong acidic medium. The colouring agents with anionic dyes are so formulated that the optimum conditions are realised for achieving the highest dyestuff penetration into hair. European patent application with laid open number EP 1 022 014 describes such compositions comprising anionic dyestuffs, solvents, as aid to enhance penetration of said dyestuffs, and a buffer solution to adjust the pH of the dyeing agent in the range from 2 to 6. For enhancing penetration of dyestuffs, solvents are used such as ethanol, benzyl alcohol, propylene carbonate, dipropylene glycol. Products are found on the professional hair dressing market applying this technology.

US 5,601,620, as well, discloses hair colouring agents with acid dyes, an organic solvent and at least one polysiloxane as a conditioner. The dyeing compositions disclosed here are having a pH in the range of 1.5 - 4.5.

Furthermore it has long been known to colour hair using direct cationic dyes and neutral nitro dyes. The direct cationic dyes developed recently and disclosed in international patent applications WO 95/01772 A1 and WO 95/15144 A1 have proven to deliver long lasting intensive colours.

Currently, these two chemical alteration processes of keratin fibres especially human hair are carried out separately in two independent processes in order to secure optimal and long lasting colouration and straightening. In practice, a person willing to have both chemical services is, therefore, asked to visit the hair dressing salon twice within a short period of time. In the first visit, hair is straightened and after a week of two or three hair is oxdatively coloured. This brings about first of all economical problems and secondly it is very much time intensive.

There are a few attempts made to carry out two chemical services in a single process, in the same hair dresser visit. For example EP 1655056 A1 discloses a process for colouring and curling hair wherein oxidative colouration is carried out first and the hair is treated with an acidic treatment and after rinsing off the acidic treatment and rolling up the hair onto curlers, a keratin reducing agent is applied onto hair and processed and finally hair is fixed with an oxidizing agent and treated with an acidic treatment again, if necessary. It has been observed that with the suggested method hair is not curled very effectively and also colours so obtained lack intensity, shine and especially durability. In the publication, nothing is disclosed on straightening keratin fibres, especially hair.

Other than the above suggested process, it has been a long hair dressing practice to use the fixing stage of a straightening process at the same time for colouring hair. It should be noted that a straightening hair involves two steps. In the first step the disulfide bonds are broken by using a reducing agent in an alkaline medium and in the second stage the broken disulfide bonds are recovered using an oxidizing agent. Oxidizing agent used in a straightening process usually comprises low level of oxidizing agent and more importantly does not have a strong alkaline pH, rather has acidic to slight alkaline pH where enough oxygen is provided to recover disulfide bonds. This is, however, quite different condition from oxidative colouring hair since there is a need for neutral to strong alkaline conditions and high concentration of oxidizing agent for securing intensive, homogeneous and long lasting colours.

Up until now it has not been known to colour and permanently shape keratin fiberes in a single process using hair direct dyes for colouring hair.

The present invention starts from the objective in achieving excellently intensive, shiny colours and at the same time excellent straightening of keratin fibres without damaging keratin fibres excessively.

It has surprisingly been found out when keratin fibres especially human hair is coloured and straightened according to a process described below, colour intensity, brilliance and durability is excellent and hair is excellently straightened. It has further been observed that hair is less damaged at the end of inventive process of the present invention, feels natural and soft and has further its natural properties such as elasticity so that hair can move freely with the movement of person's head.

A novel process for colouring and straightening hair in a single process according to the present invention includes the following steps:
a- an aqueous colouring composition based on at least one hair direct dye and not comprising any oxidative dye precursors and/or coupling substances is applied onto hair and rinsed off from hair with water after processing 5 to 45 min at a temperature of 20 to 45°C, optionally shampooed,
b- towel dried,
c- an aqueous composition comprising at least one reducing agent is applied onto hair and rinsed off from hair with water after a processing time of 5 to 60 min at ambient temperature wherein hair is continuously combed through during processing with reducing agent and towel dried,
d- optionally hair is treated with a composition comprising at least one salt and optionally rinsed off,
e- optionally a composition comprising at least one oxidizing agent is applied onto hair processed for 2 to 15 min and optionally rinsed off from hair with water and optionally towel dried, and
f- an aqueous oxidative colouring composition based on at least one hair direct dye is optionally mixed with a composition comprising at least one oxidizing agent and applied onto hair and rinsed off from hair with water after processing 5 to 45 min at a temperature of 20 to 45°C and towel dried and dried with a dryer or left to dry in the air.

Colouring composition used in step "a" of the above novel process comprises at least one hair direct dye. Suitable hair direct dyes are those of anionic, cationic and neutral nitro dyes. They can also be used in mixture.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Among those, the preferred anionic dyestuffs are Acid Red 52, Acid Violet 2, Acid Red 33, Acid Orange 4, Acid Red 27 and Acid Yellow 10 and their salts. The most preferred anionic dyes are Acid Red 52, Acid Violet 2, Acid Red 33, Acid Orange 4 and Acid Yellow 10, and their salts

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Concentration of one or more direct dyes in total is in the range of 0.001 to 5% by weight, preferably 0.01 to 4% more preferably 0.05 to 3% and most preferably 0.1 to 2.5% by weight calculated to total composition.

In another way of carrying out the invention, composition comprising at least one hair direct dye used in step "a" of the above novel process is mixed with an oxidizing composition comprising at least one oxidizing agent prior to application. As a rule, any oxidizing agent is suitable; the most preferred is hydrogen peroxide. Concentration of hydrogen peroxide in the composition is between 2 and 12% by weight calculated to the oxidizing composition prior to mixing.

Mixing ratio of composition comprising at least one hair direct dye used in step "a" of the above novel process and composition comprising at least one oxidizing agent is in the range of 3:1 to 1:3, preferably 2:1 to 1:2 and most preferably 1:1, by weight.

The aqueous colouring composition used in step "a" has a pH in the range of 2 to 12. In case that the colouring composition used in step "a" of the above novel process is mixed with a composition comprising at least one oxidizing agent, than pH of the composition after mixing of the two compositions preferably ranges between 5 and 12, more preferably 6.0 and 10.5 and most preferably 6.5 and 9.5 measured at room temperature.

Processing time of colouring composition in step "a" is preferably 5 to 30 min and more preferably 10 to 20 min.

The straightening compositions used in the process according to the invention comprise at least one reducing compound at a concentration of at least 0.5% by weight calculated to total composition. Preferred are thioglycolic acid and thiolactic acid as well as the salts thereof, in particular the ammonium and ethanolamine salts. Further useful thio compounds are in particular cysteine or the hydrochloride thereof, homocysteine, cysteamine, N-acetyl cysteine, thioglycerol, ethanediol monothioglycolate, 1,2-propyleneglycol monothioglycolate (see also WO-A 93/1791), 1,3-propanediol monothioglycolate or the isomer mixture resulting therefrom, 1,3-butanediol and 1,4-butanediol monothioglycolate and the isomer mixtures therefrom, polyethylene glycol, such as di-, tri- and tetraethyleneglycol monothioglycolates, glycerol monothiolactate and further thio acids and the esters thereof, as well as mixtures thereof.

The use of inorganic reducing sulfur compounds such as sodium hydrogen sulfite is basically also possible.

The total reduction agent content in the compositions according to the invention customarily amounts from 2.0 to 15 %, preferably 2.5 to 12.5% by weight, calculated to total or reducing composition.

The straightening compositions containing reducing agents used in step "c" of the above process can, if necessary, comprise alkalizing agents. Their quantity is dependent on the reducing agent and the desired pH-value of the composition. Reducing agent compositions preferably contain 0.1 % to 5%, in particular 0.5% to 2.5% by weight thereof, calculated to the total composition. Alkalizing agents preferred within the scope of the invention are ammonia, hydroxyalkyl amines such as monoethanol amine and triethanolamine, ammonium carbamate, ammonia and/or ammonium(bi)carbonate. It is desirable to adjust the pH-value between about 6.5 and 10.5, preferably about 7 to 9.5.

The viscosity best suited for the reducing compositions used in the above processes step "c" depends very much on the nature of the process. In the first process described above, wherein hair is fastened on a hard surface with aid of composition comprising at least one reducing agent, comparatively higher viscous compositions are preferred. Viscosity of such compositions are typically 5,000 mPa.s or more, preferably 7,500 mPa.s or more, more preferably 10,000 mPa.s or more measured at 20°C in a Brookfield viscosimeter with an appropriate spindle and rotation (i.e. spindle no. 5 at 5 rpm). The reducing composition described above is certainly suitable for the other two processes described above. However, for these two processes viscosity is not very critical so the compositions can have lower viscosity values. In the preferred embodiment such compositions should preferably have viscosity values in the range of 500 to 10,000 mPa.s, more preferably 750 to 7,500 m.Pa.s and most preferably 1,000 to 5,000 mPa.s, measured at 20°C with Brookfield viscosimeter with an appropriate spindle and rotation (i.e. spindle no. 5 at 5 rpm).

The viscosity is adjusted by addition of the appropriate amounts of thickening agents known per se, such as cellulose derivatives. Thickening may as well be realized by formulating a composition in form of an emulsion with the use of C₁₀-C₂₂-fatty alcohols, in admixture with long mono alkyl chain quaternary ammonium surfactants.

Compositions of step "d" according to the processes disclosed above have the function of deswelling hair and therefore they comprise at least one salt preferably in a concentration range from 0.5 to 15%, more preferably 1 to 12.5% and most preferably 2 to 12.5% by weight calculated to total composition. In the process
wherein hot iron is used, the composition has at the same time the function of thermo protection and comprises at least one oil and/or oily compound.

In principal any water soluble salt is suitable for the purpose of the composition of step "d". Preferably salt is an inorganic substance. In the preferred embodiment, salts are preferably selected from salts of mono or divalent cations with mono and divalent anions. Preferred cations are sodium, calcium, potassium and magnesium and anions are chloride and sulfate. Suitable ones are such as sodium chloride, sodium sulfate, magnesium sulfate, potassium chloride, potassium sulfate, magnesium chloride, calcium chloride, ammonium salts such as ammonium chloride and ammonium sulfate. Preferred salts are sodium chloride, sodium sulfate, magnesium sulfate, potassium chloride, potassium sulfate, magnesium chloride, and calcium chloride. More preferably the salts are sodium chloride, sodium sulfate, magnesium sulfate, potassium chloride, potassium sulfate and magnesium chloride. In particular, with magnesium sulfate and sodium chloride exceptionally good results were observed.

Composition used in step "d" in combination with hot iron comprise at least one oil and/or oily compound at a concentration of 10 to 100%, preferably 15 to 90%, more preferably 20 to 80% and most preferably 25 to 70% by weight calculated to total composition.

Suitable oils are selected from natural and synthetic oily. Natural oils are vegetable triglycerides such as avocado oil, olive oil, almond oil, peach oil, passiflora oil, black cumin oil, borage oils, evening primrose oil, grapeseed oil, hempseed oil, kukui nut oil, rosehip oil, safflower oil, walnut oil and weatgerm oil. Natural oil is preferably used at a concentration of 0.1 to 5% by weight calculated to total composition.

Among synthetic oils, fatty acid fatty alcohol esters are suitable according to the formula

R₇ C (O) O R₈

wherein R₇ is saturated or unsaturated, branched or straight alkyl chain with 7-23 C atoms and R₈ is saturated or unsaturated, branched or straight alkyl chain with 1 to 24 C atoms. Preferably, R₈ is 1 to 12, more preferably 1 to 8 and most preferably 1 to 4 C atoms.

Suitable examples are behenyl behenate, behenyl isostearte, butyl stearate, butyl oleate, butyl myristate,butyloctyl oleate,cetyl palmitate, cetyl myristate, cetyl oleate, cetyl caprylate, cetyl caprate, decyl oleate, decyl cocoate, decyl isostearate, ethylhexyl myristate, ethyl hexyl laurate, ethyl hexyl oleate, ethyl isostearte, ethyl laurate, ethyl linoleate, ethyl myristate, ethyl oleate, ethyl palmitate, ethylricinoleate, ethyl stearate, hexyl isostearet, hexyl laurate, hexyl myristate, hexyl stearate, hexyl decyl oleate, isobutyl laurate, isobutyl myristate, isobutyl palmitate, isobutyl stearate, isocetyl behenate, isobutyl laurate, isobutyl oleate, isobutyl stearate, isobutyl cocoate, isohexyl caprate, isopropyl palmitate, isopropyl stearate, isopropyl behenate, isopropyl laurate, isopropyl oleate, isopropyl ricinoleate and isopropyl palmitate.

Fatty acid fatty alcohol esters are present at a concentration of 0.1 to 10% by weight calculated to total composition.

Among synthetic oils silicones are the preferred ones and can be used alone or in combination with the above mention natural oils and/or fatty acid fatty alcohol esters. Silicones suitable are volatile and non-volatile ones and volatile ones are preferred and can be sued alone or in combination with one or more other type of silicones such as arylated silicones and high viscosity and/or high molecular weight silicones. Volatile silicones are dimethicones with low viscosity such as 0.65 cSt or 1 cSt, and cyclic silicones such as cycloheptasiloxane, cyclohexasiloxane, cyclopentasiloxane, cyclotetrasiloxane, cyclotrisiloxane and cyclomethicone. Nonvolatile ones are dimthicones with high viscosity commercially available from Dow Corning with the trade name DC 200 with various viscosity and as well as arylated silicones such as phenyl methicone, phenyl trimethicone, diphenyl dimethicone, diphenylsiloxy phenyl trimethicone, tetramethyl tetraphenyl trisiloxane, triphenyl trimethicone, tetramethly tetraphenyl trisiloxane and trimethyl pentaphenyl trisiloxane.

Silicones are comprises at a concentration of 10 to 100%, preferably 15 to 90%, more preferably 20 to 80% and most preferably 25 to 70% by weight calculated to total composition. In another preferred from more than 50% by weight of the above mentioned concentration must be volatile silicone.

Composition comprising at least one oxidizing agent used in step "e" of the above novel processes comprises preferably hydrogen peroxide as an oxidizing agent at a concentration of preferably 1 to 5%, more preferably 2 to 3% by weight calculated to total composition. The pH of the composition is in the range of 2 to 6, preferably 2.5 to 5, more preferably 3 to 5 and most preferably 3 to 4.5 measured at room temperature.

In the novel process of the present invention, aqueous colouring compositions used in step "f" can basically be the same composition used in step "a". Here again in another way of carrying out the invention, colouring composition is mixed with a composition comprising at least one oxidizing agent preferably hydrogen peroxide. Oxidizing composition used here is same as the one used in step a.

The aqueous colouring composition used in step "f" has a pH in the range of 2 to 12, same as the one used in step "a". In case that the colouring composition used in step "a" of the above novel process is mixed with a composition comprising at least one oxidizing agent, than pH of the composition after mixing of the two compositions preferably ranges between 5 and 12, more preferably 6.0 and 10.5 and most preferably 6.5 and 9.5 measured at room temperature.

If a further embodiment of the present invention aqueous colouring composition used in step "f" comprises further at least one oxidative dyestuff precursor and optionally at least one coupling agent. In this case, the composition must be mixed with a composition comprising at least one oxidizing agent and should have pH in the range of 5 to 12, more preferably 6.0 to 10.5 and most preferably 6.5 to 9.5 measured at room temperature and after mixing the two compositions.

As a rule any oxidative dye precursor is suitable for the purpose of the present invention. Examples to suitable oxidative dye precursors are p-phenylenediamine, p-methylaminophenol and substituted p-phenylenediamines such as 2,5-diaminotoluene, 2-n-propyl or 2-ethyl-p-phenylenediamine, 2,6-di-methyl-p-phenylenediamine, 2-(2,5-diaminophenyl) ethanol, 1-amino-4-bis-(2'-hydroxy-ethyl)aminobenzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, pyrazole and the derivatives thereof such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diaminophenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene or the water-soluble salts thereof.

Further suitable aminopyridines are 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxypyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof.

Further, Indole and indoline derivatives can as well be contained in the colouring composition of the present invention. Examples to those are: 6-aminoindol, 6-hydroxyindole, 1-ethyl-6-hydroxyindole, 1-methyl-4-hydroxyindol, 1-methyl-6-hydroxyindole, 2-methyl-6-hydroxyindole, 5-hydroxyindol, 4-hydroxyindol, 5,6-dihydroxyindole, 6-aminoindoline, 6-hydroxyindoline, 1-ethyl-6-hydroxyindoline, 1-methyl-4-hydroxyindoline, 1-methyl-6-hydroxyindoline, 2-methyl-6-hydroxyindoline, 5-hydroxyindoline, 4-hydroxyindoline, 5,6-dihydroxyindoline and their respective salts.

Further to the oxidative dye precursors, aqueous colouring composition applied onto hair in step "f" comprises at least one coupling agent. In general any coupling agent known in the art is suitable for the purpose of the present invention.

Suitable coupling agents are resorcinol, 2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxy-pyridine, 2-dimethyl-amino-5-aminopyridine, 2,6-diaminopyridine, 1,3-diamino- benzene, 1-amino-3-(2'-hydroxyethylamino)benzene, 1-amino-3-[bis(2'-hydroxy-ethyl) amino]benzene, α -naphthol, 4,6-dichlororesorcinol, 1,3-diamino-toluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof. 5-amino-2-methylphenol, 2-methyl-5-hydroxyethylaminophenol, 2,4-diamnophenoxyethanol, 2-amino-4-hydroxyethylaminoanisol, 2-methyl-5-amino-6-chlorophenol, 1,3-bis(2,4-diaminophenoxy)propane, 2-bis(2-hydroxyethyl)aminotoluene, 2-amino-5-methylphenol or the water-soluble salts thereof.

Concentration of oxidation dyes precursors and coupler is customarily in the range of 0.01 to 5% by weight calculated to total composition prior to mixing.

Either oxidative colouring compositions used in steps "a" and "f" and/or reducing composition used in step "c" and/or the composition of step "d" and/or oxidizing composition used in steps "a" "e" and "f" can comprise the following ingredients explained in detail below, unless otherwise stated.

The above mentioned compositions comprise surfactants selected from anionic, nonionic, cationic and amphoteric ones. Their proportion ranges from 0.05 % to 10%, in particular from 0.1 % to 5 % by weight, calculated to total composition.

Suitable anionic surfactants are especially the known alkyl ether sulfates and carboxylic acids, in particular in form of their alkali salts, as well as protein fatty acid condensates.

Suitable nonionic surfactants, which are preferred within the scope of the invention, are in particular C₈-C₁₈-fatty alcohol polyglycol ethers, fatty acid polyglycol esters, fatty acid alkanolamides, amineoxides, and especially C₈-C₁₈-alkyl polyglucosides.

Also possible is the incorporation of amphoteric surfactants, such as the known alkyl betaines, alkyl amido betaines, and alkyl amphoacetates.

Further according to a further preferred embodiment, the above mentioned compositions comprise at least one cationic surfactant according to general formula 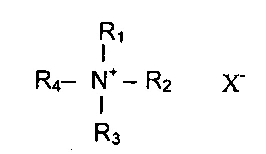 where R₁ is a saturated or unsaturated, branched or non-branched alkyl chain with 8-22 C atoms or

R₅CO NH (CH₂)ₙ

where R₅ is saturated or unsaturated, branched or non-branched alkyl chain with 7-21 C atoms and n has typical value of 0 - 4 or

R₆CO O (CH₂)ₙ

where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-22 C atoms or

R₅CO NH (CH₂)ₙ

where R₅ is saturated or unsaturated, branched or non-branched alkyl chain with 7-21 C atoms and n has typical value of 0 - 4 or

R₆ CO O (CH₂)ₙ

where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms, and X is chloride, bromide or methosulfate.

Concentration of cationic surfactant is in the range from 0.05 % to 5 %, preferably 0.1 % to 2.5 % by weight, calculated to total composition.

Suitable long-chain quaternary ammonium compounds which can be used alone or in admixture are in particular cetyl trimethyl ammonium chloride, dimethyl dicetyl ammonium chloride, trimethyl cetyl ammonium bromide chloride, stearyl trimethyl ammonium chloride, dimethyl stearyl benzyl ammonium chloride, benzyl tetradecyl dimethyl ammonium chloride, dimethyl dihydrogenated tallow ammonium chloride, lauryl pyridinium chloride, lauryl dimethyl benzyl ammonium chloride, lauryl trimethyl ammonium chloride, tris-(oligooxyethyl) alkyl ammonium phosphate, cetyl pyridinium chloride, etc.

Additional examples to so called ester and amido quaternary ammonium compounds are distearyldimonium chloride, dipalmitoylethylhydroxyethylmonium chloride, dioleoylethyl dimethyl ammonium methosulfate, dioleoylethyl hydroxyethylmonium methosulfate, dilinolamidopropyldimonium chloride, dioleylethyl hydroxyethylmonium chloride, dipalmitoylethyldimonium chloride.

From the above quaternary ammonium compounds disclosed with the general formula, especially to mention are those compounds known per se and are on the market, for example, under the trade names "Schercoquat^{®}", "Dehyquart^{®} F30" and "Tetranyl^{®}". Use of these compounds, the so-called "esterquats", in hair care compositions is described, for example, in WO-A 93/107 48, WO-A 92/068 99 and WO-A 94/166 77, wherein, however, there is no reference made to the combinations according to the present invention and the advantageous properties thereof.

Again from the above quaternary ammonium compounds disclosed with the general formula, especially to mention are those compounds are known per se and on the market, for example, under the trade name "INCROQUAT^{®} HO" or "OCS". These compounds are known with a general ingredient category under "amidoquat" in the cosmetic industry.

Compositions mentioned above can also comprise thickening agents to adjust the viscosity to the desired value. All known thickening agents such as anionic, non-ionic, cationic polymers are suitable for the purpose of the invention. It should be noted that compatibility with various ingredients of individual compositions should be paid attention when selecting thickening polymer. Suitable ones are cellulose derivatives such as hydroxyethyl or methyl cellulose, anionic acrylate polymers, cationic cellulose derivatives.

Thickening of the compositions can also be achieved by formulating an emulsion. In such a case at least one fatty alcohol with an alkyl chain length of 12 to 22 C atoms should be present in the composition. Examples are cetyl alcohol, stearyl alcohol or their mixture, myristyl alcohol and behenyl alcohol. Branched fatty alcohols such as octyldodecanol may also be present either alone or in mixture with linear fatty alcohols. Emulsions must also comprise an emulsifier selected from anionic, non-ionic and cationic surfactants as mentioned above. Most preferred emulsifiers are those ethoxylated fatty alcohols as nonionic ones, alkyl sulfates or alkyl ether sulfates as anionc ones and monoalkyl quaternary ammonium ones as cationic surfactants.

Further the above mentioned compositions may comprise additional cationic polymer. Basically suitable are all cationic polymers listed under the generic name "Polyquaternium" in the CTFA International Cosmetic Ingredient Dictionary.
Examples are Polyquaternium 6, Polyquaternium 7, Polyquaternium 10, Polyquaternium 11, Polyquaternium 22 and Polyquaternium 28, Polyquaternium 39, Polyquaternium 16 and Polyquaternium 87.

In particular and in further preferred embodiment of the present invention, aqueous reducing composition used in step "c" of the novel process comprises further at least one copolymer of vinylpyrrolidone and quaternized vinylimidazole at a concentration in the range of 0.1 to 2.5% by weight, calculated to total composition. Preferred copolymers of vinylpyrrolidone and quaternized vinylimidazole has a charge density of at least 2 meq/g, preferably 3.0 meq/g, more preferably 6.1 meq/g at pH 7.0. Such polymers are available under the trade name Luviquat from BASF.

The cationic polymers also include the quaternized products of graft polymers from organopolysiloxanes and polyethyl oxazolines described in EP-A 524 612 and EP-A 640 643. Such polymer is known with its CTFA name Polysilicone-9.

Concentration of one or more additional cationic polymers is in the range from 0.05 % to 2.5 %, preferably 0.1 % to 1.5 % by weight, calculated to total composition.

Further the above mentioned composition comprise preferably at least one organic solvent. Suitable organic solvents are 2-methyl-1,3-propanediol, mono and dialcohols or the ethers thereof, in particular mono-C₁-C₃-alkyl ether, ethanol, n-propanol, isopropyl alcohol, 1-methoxypropanol, 1-ethoxypropanol and ethoxydiglycol, diols and their esters 1,3- and 1,4-butanediol, diethyleneglycol and the monomethyl and monoethyl ether thereof, dipropylene glycol and the monomethyl and monoethyl ether thereof, glycerol, hexanetriol, ethyl carbitol, benzyl alcohol, benzyloxy ethanol, propylene carbonate, N-alkyl pyrrolidone, and urea or their mixture preferably in an amount from about 0.1 % to 10 % by weight, calculated to the total composition.

The above mentioned compositions can comprise further ceramide type of compound such as cetyl-PG-hydroxyethylpalmitamide.

Further optional ingredient are sterols, especially the phytosterols are useful hair restructuring compounds can be present in the above mentioned compositions. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

Optionally fatty acids of C10 to C22 may be incorporated into the compositions of the present invention at a concentration of preferably 0.01 to 10%, preferably 0.1 to 5 and more preferably 0.2 to 2.5% by weight calculated to total composition.

In a further preferred embodiment of the present invention, intermediate treatment composition comprises at least one diamine compound. Preferred diamide compounds are according to the general structure 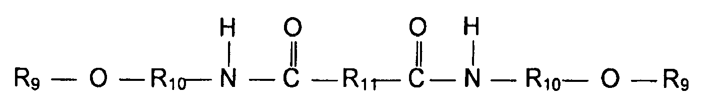 wherein R₉ is a linear or branched, saturated or unsaturated alkyl chain with 1 to 12 C atoms which may be substituted with hydroxy and/or alkoxy groups, preferably R₉ is linear or branched, saturated or unsaturated alkyl chain with 1 to 12 C atoms which may be substituted by 1 to 3 substituents selected from a hydroxy group and C1 to C6 alkoxy group, more preferably R₉ is a unsubstituted alkyl group with 1 to 12 C atoms, and alkyl group with 2 to 12 C atoms substituted by one or two hydroxyl groups, by one alkoxy group with 1 to 6 C atoms or by one hydroxyl and one alkoxy group with 2 to 6 C atoms, R₁₀ is linear or branched alkyl chain with 1 to 5 C atoms, preferably linear or branched alkyl chain with 2 to 5 C atoms and more preferably an alkyl chain with 2 to 3 C atoms, and R₁₁ linear or branched, saturated or unsaturated alkyl chain with 1 to 22 C atoms, preferably linear or branched, saturated or unsaturated alkyl chain with 11 to 22 C atoms.

Preferred individual diamide compounds are the ones according to the formula A to G.

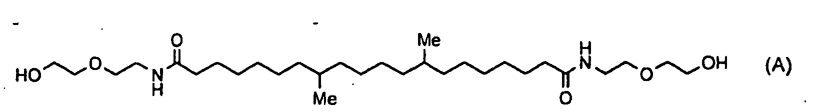

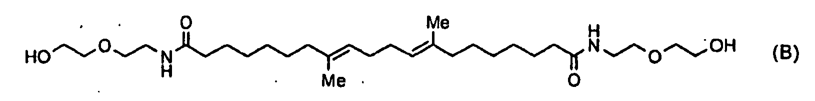

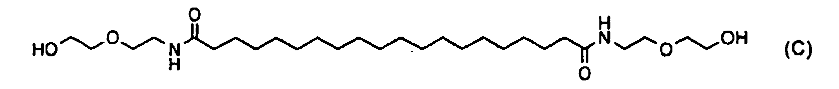

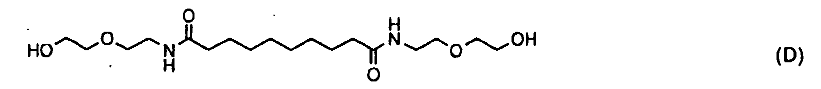

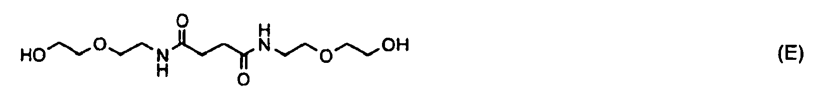

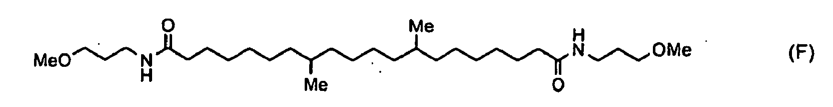

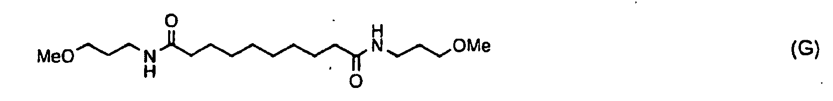

Particularly preferred diamide compound is the compound F which is bis (methoxypropylamido) isodocosane and commercially available from Kao Corporation - Japan.

Concentration of diamide compounds in the intermediate treatment compositions of the present invention is in the range of 0.001 to 5%, preferably 0.002 to 3% more preferably 0.005 to 2% and most preferably 0.01 to 1% by weight calculated to total of each composition.

It is the preferred embodiment of the present invention that oxidative colouring compositions used in steps "a" and "e" of the novel process comprise at least one saturated or unsaturated fatty acid with 12 to 22 C atoms in its molecule at a concentration of 0.1 to 10%, preferably 0.1 to 5% by weight, calculated to total composition.

Another preferred compound in the compositions mentioned above used in the novel process of the present invention is silicone compounds and especially aminated silicones such as amodimethicone available from for example Dow Corning under the brand names Dow Corning 949 Emulsion and Dow Corning 2-8194 ME. Concentration of silicones, especially amodimethicone, is in the range of 0.05 to 2.5%, preferably 0.1 to 1% by weight calculated to total composition.

Additionally, one or more natural oil component may be incorporated into the above mentioned compositions. Suitable are such as olive oil, almond oil, avocado oil, wheatgerm oil, ricinus oil or their mixture. Concentration of these natural oil ingredients should be 0.01 to 2.5%, preferably 0.01.to 1%, more preferably 0.05 to 0.5% by weight, calculated to total composition.

Further additional compounds may be present in the above mentioned compositions of the present invention is ubichinone of the formula 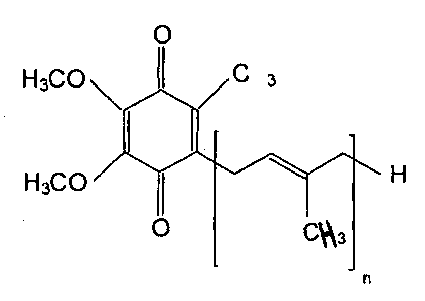 where n is a number between 1 and 10. Preferred ubichinones are the ones where n is a number between 6 and 10 and especially preferred is Ubichinone 50 where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

The compositions used according to the invention can naturally comprise all substances customarily found is such compositions such as fragrance, chelating agent, reducing agent in the oxidative colouring compositions for stabilizing oxidation dyes during storage, preservatives, acids or alkaline compounds used for adjusting pH, foam preventing agent such as silicones and especially simethicone.

The following examples are to illustrate but not to limit the invention.

### Example 1

### Colouring composition for step "a" and "e"

| | % by weight |
|---|---|
| Ext. Violet 2 (Cl 60730) | 0.32 |
| Orange 4 (Cl 15510) | 0.25 |
| Red 33 (Cl 17200) | 0.2 |
| Yellow 10 (Cl 47005) | 0.2 |
| Hydroxypropylated sachharadies | 1.5 |
| Propylene carbonate | 25 |
| Lactic acid | q.s. to pH 2.0 |
| Fragrance, preservative | q.s. |
| Water | to 100 |

### Reducing composition for step "c"

| | % by weight |
|---|---|
| Cetearyl alcohol | 4.5 |
| Steareth-2 | 2.0 |
| Behentrimonium chloride | 1.5 |
| PEG-9 dimethicone | 0.5 |
| Mineral oil | 1.0 |
| Isopropyl palmitate | 1.0 |
| Ammonium thioglycolate (70%) | 3.2 |
| Ammonium hydrogen carbonate | 2.0 |
| Amodimethicone | 0.5 |
| PEG-40-Hydrogenated castor oil | 0.7 |
| Perfume | 0.4 |
| Ethanolamine | 2.3 |
| EDTA | 0.2 |
| Water | ad 100.0 |

The composition has a viscosity of approximately 18,000 mPa.s measured with Brookfield viscosimeter with Spindle 5 at 10 rpm. pH of the composition was 8.3.

### Composition of step "d"

| | % by weight |
|---|---|
| Magnesium sulphate | 10.0 |
| Asparagic acid | 0.25 |
| Alanin | 0.5 |
| Cetrimonium chloride | 1.0 |
| Hydroxyethylcellulose | 0.25 |
| Citric acid | q.s. to pH 4.10 |
| Water | q.s. to 100 |

### Oxidizing agent for step step"e"

| | % by weight |
|---|---|
| Hydrogen peroxide | 2.5% |
| Phosphoric acid | q.s. to pH 3.5 |
| Acrylate copolymer | 0.25 |
| Water | to 100 |

Hair of a female volunteer having approximately shoulder length natural curly hair was coloured and straightened using above compositions. The process was carried out as follows - as described as the first process above and in claim 1.

First of all, colouring composition of step "a" was applied onto hair and processed for 20 min at approximately 40°C and rinsed off from hair with water and the hair was towel dried. Afterwards, reducing composition of step "c" was applied onto hair and hair was fixed in the straight form on an aluminium sheet surface and processed for approximately 20 min at ambient temperature and rinsed off with water. Subsequently, composition of step "d" was applied and processed for 10 min and without rinsing off oxidizing composition of step "e" was applied onto hair. After processing of additional 10 min, colouring composition of step "f" was applied onto hair. After processing approximately 30 min at 40°C hair was rinsed off with water and dried.

It was observed that hair was excellently straightened and had an intensive brown shade.

For comparative purposes, the above process was carried out wherein the step "a", the first step, was omitted. It was observed that colour intensity was reduced and the homogeneity of the colour was also not optimum.

### Example 2

### Colouring composition for step "a"

| Stearyl alcohol | 12.0 (% by wt.) |
|---|---|
| Stearamide MEA | 4.0 |
| Cocamide MEA | 2.0 |
| Propylene glycol stearate SE | 4.0 |
| Sodium lauryl sulfate | 0.3 |
| Oleic acid | 2.0 |
| 1,2-Propanediol | 1.5 |
| Na-EDTA | 0.5 |
| Sodium sulfite | 1.0 |
| Ammonium bicarbonate | 0.95 |
| Ascorbic acid | 0.2 |
| Perfume | 0.4 |
| Ammonia, 25% | 8.0 |
| Ammonium chloride | 0.5 |
| Polysilicone-9 | 0.15 |
| Coenzyme Q10 | 0.001 |
| Basic red 51 | 0.5 |
| Water | ad 100.00 |

The pH of the above composition is approximately 10.5.

### Oxidizing agent for step "a"

| | % by weight |
|---|---|
| Hydrogen peroxide | 6% |
| Phosphoric acid | q.s. to pH 2.5 |
| Simethicone | 0.1 |
| Acrylate copolymer | 0.25 |
| Water | to 100 |

The above compositions were mixed at a weight ratio of 2:1 (oxidative dye comprising composition and oxidizing composition) and the mixture had a pH of 9.5.

### Oxidative colouring composition for step "e"

| Stearyl alcohol | 12.0 (% by wt.) |
|---|---|
| Stearamide MEA | 4.0 |
| Cocamide MEA | 2.0 |
| Propylene glycol stearate SE | 4.0 |
| Sodium lauryl sulfate | 0.3 |
| Oleic acid | 2.0 |
| 1,2-Propanediol | 1.5 |
| Na-EDTA | 0.5 |
| Sodium sulfite | 1.0 |
| Ammonium bicarbonate | 0.95 |
| Ascorbic acid | 0.2 |
| Perfume | 0.4 |
| Ammonia, 25% | 1.0 |
| Ammonium chloride | 0.5 |
| Polysilicone-9 | 0.15 |
| Coenzyme Q10 | 0.001 |
| 2,5-diaminotoluene sulphate | 0.1 |
| Tetraaminopyrimidine sulphate | 2.2 |
| 2-methyl-5-hydroxyethylaminophenol | 0.1 |
| 2- methylresorcinol | 1.0 |
| 1-naphtol | 0.1 |
| Acid red 52 | 1.5 |
| Water | ad 100.00 |

The pH of the above composition is approximately 10.5 and after mixing with oxidizing agent at a weight ratio of 1 to 1 had a pH of 6.8.

Using the above composition hair was coloured and straightened as follows: First, colouring composition comprising Basic red 51 was mixed with oxidizing composition for step "a" given above and applied onto hair and rinsed off with water after processing for 20 min at ambient temperature and towel dried. Afterwards, reducing composition of step "c" of Example 1 was applied onto hair and processed for approximately 20 min with continuous combing through at ambient temperature and rinsed off with water. Subsequently, composition of step "d" was applied and processed for 10 min and without rinsing off oxidizing composition of step "e" was applied onto hair. After processing of additional 10 min, colouring composition of step "f" was applied onto hair. After processing approximately 30 min at 40°C hair was rinsed off with water and dried.

At the end, it was observed that hair is coloured intensive red-violett and had excellent straight appearance.

For comparative purposed the above process was carried out wherein step "a", the first step" was omitted. It was observed that colour intensity is reduced and homogeneity of the colour was also not optimum.

Furthermore, again for the comparative purpose the step f was omitted from the above process and it was observed that the red intensity decreased significantly and hair was not homogeneously coloured.

### Example 3

Example 2 was repeated wherein following dyestuffs were used in step "a" and step "e".

### Dyestuff composition for step "a"

| | % by weight |
|---|---|
| 4-hydroxypropylamino-3-nitrophenol | 2.5 |
| 2-amino-6-chloro-4-nitrophenol | 0.5 |
| HC Red No.3 | 0.2 |

### Dyestuff composition for step "e"

| | % by weight |
|---|---|
| Tetraaminopyrimidine sulphate | 2.5 |
| 2-amino-6-chloro-4-nitrophenol | 0.4 |
| Acid Red 52 | 0.9 |
| 2-methylresorcine | 1.3 |

Same process as used in example 2 was used.

It was observed that hair was coloured into shiny and intensive red copper direction and had excellent curl appearance and elasticity.

The example 1 as given above was repeated with the same compositions except the step d was skipped (not used).

Here again intensive shiny natural black colour was obtained and hair had excellent straightened appearance.

### Example 4

With the compositions of Example 1, hair was straightened and coloured according to the process of claim 3. Additionally, the following composition is used in step "d".

### Composition for step "d"

| | % by weight |
|---|---|
| Dimethicone | 25 |
| Phenyltrimethicone | 1 |
| Avocado oil | 1 |
| Isopropylpalmitate | 1 |
| Cetrimonium chloride | 0.2 |
| Amodimethicone | 0.5 |
| Citric acid | q.s. to pH 5.0 |
| Water | q.s. to 100 |

The above composition when prepared by mixing all the components is a two phase composition and must be shaken to homogeneity before application onto hair.

### Reducing composition for step "c"

| Ammonium thioglycolate (60%) | 21.3 (% by wt. |
|---|---|
| Ammonium hydrogen carbonate | 5.0 |
| 1,3- butylene gylcol | 3.0 |
| Polyquaternium-16* | 0.5 |
| PEG-40-Hydrogenated castor oil | 0.7 |
| Perfume | 0.4 |
| Ammonia, 25% | ad pH 8.3 |
| Water | ad 100.0 |

First of all, colouring composition of step "a" was applied onto hair and processed for 20 min at approximately 40°C and rinsed off from hair with water and the hair was towel dried. Afterwards, reducing composition of step "c" (above) was applied onto hair and processed for approximately 20 min at ambient temperature and rinsed off with water. Subsequently, composition of step "d" was applied and subsequently, a hot iron suitable for straightening hair and commercially available was used to straighten hair at a temperature of approximately 140°C. The hot iron had round shaped ironing metal parts. It was also observed in an additional trial a flat shaped hot iron can be used in the same way. This was followed by application of oxidizing composition of step "e" and processing of additional 10 min. Colouring composition of step "f" was applied onto hair. After processing approximately 30 min at 40°C hair was rinsed off with water and dried.

It was observed that hair was excellently straightened and had an intensive brown shade.

For comparative purposes, the above process was carried out wherein the step "a", the first step was omitted. It was observed that colour intensity was reduced and the homogeneity of the colour was also not optimum.

## Claims

1. Process for colouring and straightening hair in a single process **characterised in that** it comprises the following steps
a- an aqueous colouring composition based on at least hair direct dye but not comprising oxidative dye precursor and coupling substances is applied onto hair and rinsed off from hair with water after processing 5 to 45 min at a temperature of 20 to 45°C, optionally shampooed,
b- towel dried,
c- an aqueous composition comprising at least one reducing agent is applied
onto hair and rinsed off from hair with water after a processing time of 5 to 60 min at ambient temperature wherein hair is continuously combed
through during processing with reducing agent and towel dried,
d- optionally hair is treated with a composition comprising at least one salt
and optionally rinsed off,
e- a composition comprising at least one oxidizing agent is applied
onto hair processed for 2 to 15 min and optionally rinsed off from hair with water and optionally towel dried, and
f- an aqueous colouring composition based on at least one hair direct dye is optionally mixed with a composition comprising at least one oxidizing agent and applied onto hair and rinsed off from hair with water after processing 5 to 45 min at a temperature of 20 to 45°C and towel dried and dried with a dryer or left to dry in the air.

2. - Process according to claim 1 **characterised in that** colouring compositions has a pH from 2 to 12 and at least one hair direct dye is selected from anionic, cationic and neutral nitro dyes or their mixtures.

3. - Process according to claims 1 and 2 **characterised in that** colouring composition in step "a" is mixed with a composition comprising at least one oxidizing agent and has pH from 5 to 12 after mixing.

4. - Process according to claims1 to 3 **characterised in that** reducing composition of step "c" comprises at least one reducing agent selected from thioglycolic acid, thiolactic acid and/or their salts in particular ammonium and ethanolamine salts, cystein and/or hydrochloride salt thereof, homocysteine, cysteamine, N-acetyl cysteine, thioglycerol, ethanediol monothioglycolate, 1,2-propyleneglycol monothioglycolate, 1,3-propanediol monothioglycolate or the isomer mixture resulting therefrom,1 ,4-butanediol monothioglycolate and the isomer mixtures therefrom, polyethylene glycol, such as di-, tri- and tetraethyleneglycol monothioglycolates, glycerol monothiolactate and further thio acids and the esters thereof, as well as mixtures thereof at a concentration of 2 to 15%, by weight, calculated to total composition.

5. - Process according to any of the preceding claims **characterised in that** reducing composition at step "c" has a pH from 6.5 to 10.5.

6. - Process according to any of the preceding claims **characterised in that** the composition used in step "d" comprises at least one oil selected from natural oils, fatty acid fatty alcohol esters and silicone oils, preferably it comprises at least one volatile silicone oil and at least one non-volatile silicone oil.

7. - Process according to any of the preceding claims **characterised in that** the oxidizing agent used in step "e" comprises at least one oxidizing agent at a concentration 1 to 5% by weight, calculated to total composition and has a pH from 2 to 6.

8. - Process according to any of the preceding claims **characterised in that** oxidative colouring composition of step "f' comprises at least one oxidative dye precursor and optionally at least one coupling agent.

9. - Process according to any of the preceding claims **characterised in that** any of the compositions used in any step of the process comprises a thickening agent selected from anionic, non-ionic and cationic polymers.

10. - Process according to any of the preceding claims **characterised in that** any of the compositions used in any step of the process is an emulsion and comprises at least fatty alcohol with an alkyl chain length of 12 to 22 C atoms.

11. - Process according to any of the preceding claims **characterised in that** any of the compositions used in any step of the process comprises a surfactant selected from anionic, cationic, non-ionic and amphoteric ones, wherein cationic surfactant is preferably selected from compounds of formula 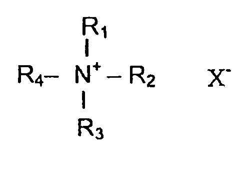 where R₁ is a saturated or unsaturated, branched or non-branched alkyl chain with 8-22 C atoms or
R₅CO NH (CH₂)ₙ
where R₅ is saturated or unsaturated, branched or non-branched alkyl chain with 7-21 C atoms and n has typical value of 0 - 4 or
R₆CO O (CH₂)ₙ
where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-22 C atoms or
R₅CO NH (CH₂)ₙ
where R₅ is saturated or unsaturated, branched or non-branched alkyl chain with 7-21 C atoms and n has typical value of 0 - 4 or
R₆ CO O (CH₂)ₙ
where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms, and X is chloride, bromide or methosulfate.

12. - Process according to any of the preceding claims **characterised in that** any of the compositions used in any step of the process comprises one or more of compounds selected from organic solvent, silicone compound, fatty acid with 12 to 22 C atoms, cationic polymer and ubichinone of the formula 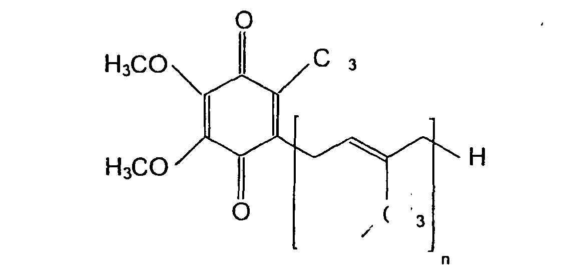 where n is a number between 1 and 10.

## Patentansprüche

1. Verfahren zum Färben und Glätten von Haaren in einem Prozess, **dadurch gekennzeichnet, dass** es folgende Schritte enthält:
a- eine wässrige färbende Zusammensetzung, basierend auf mindestens einen direkt
ziehende Haarfarbstoff, die aber keine oxidativen Farbentwickler und Kupplersubstanzen enthält, wird auf das Haar aufgetragen und mit Wasser nach einer Einwirkzeit von 5 bis 45 Minuten bei einer Temperatur bei 20 bis 45°Caus dem Haar ausgespült, optional shampooniert,
b- Handtuch getrocknet,
c- eine wässrige Zusammensetzung die mindestens ein Reduktionsmittel enthält wird
auf das Haar aufgetragen und mit Wasser nach einer Reaktionszeit von 5 bis 60 Minuten bei Raumtemperatur ausgespült und mit dem Handtuch getrocknet, wobei das Haar während der Einwirkzeit mit dem Reduktionsmittel kontinuierlich durchgekämmt wird.
d- optional wird das Haar mit einer Zusammensetzung die mindestens ein Salz enthält
behandelt und optional ausgespült,
e- eine Zusammensetzung die mindestens ein Oxydationsmittel enthält wird auf das
Haar aufgetragen, 2 bis 15 Minuten einwirken lassen und optional aus dem Haar mit Wasser ausgespült und optional mit dem Handtuch getrocknet und,
f- eine wässrige färbende Zusammensetzung, basierend auf mindestens einen direkt
ziehenden Haarfarbstoff wird optional mit einer Zusammensetzung die mindestens ein Oxydationsmittel enthält gemischt und auf das Haar aufgetragen und nach einer Einwirkzeit von 5 bis 45 Minuten bei einer Temperatur von 20 bis 45°C ausgespült, mit dem Handtuch getrocknet und mit einem Fön getrocknet oder an der Luft getrocknet.

2. Verfahren nach Anspruch 1, **dadurch gekennzeichnet, dass** die Zusammensetzung einen pH- Wert von 2 bis 12 hat und mindestens einen direkt ziehenden Haarfarbstoff, ausgewählt aus anionischen-, kationischen- und neutralen Nitrofarbstoffen oder deren Mischungen enthält.

3. Verfahren nach den Ansprüchen 1 und 2, **dadurch gekennzeichnet, dass** die Zusammensetzung in Schritt "a" mit einer Zusammensetzung gemischt wird, die mindestens ein Oxydationsmittel enthält und nach dem Mischen einen pH von 5 bis 12 hat.

4. Verfahren nach den Ansprüchen 1 bis 3, **dadurch gekennzeichnet, dass** die reduzierende Zusammensetzung von Schritt "c" mindestens ein Reduktionsmittel, ausgewählt aus Thioglykolsäure, Thiomilchsäure und/oder deren Salze im Speziellen Ammonium- und Ethanolaminsalze, Cystein und/oder Hydrochloridsalze davon, Homocysteine, Cysteamin, N-Acetylcystein, Thioglycerol, Ethandiolmonothioglykolat, 1,2-Propyleneglykolmonothioglycolat, 1,3-Propandiolmonothioglycolat oder die daraus resultierende Isomermischung, 1,4-Butanediolmonothioglycolat und deren Isomermischung, Polyethylen glykol wie Di-, Tri- und Tetraethylenglykolmonothioglykolate, Glycerolmonothiolactat und weiter Thiosäuren und deren Ester als auch Mischungen davon in einer Menge von 2 bis 15 Gew.- %, berechnet auf die Gesamtzusammensetzung, enthält.

5. Verfahren nach einem der vorhergehenden Ansprüche, **dadurch gekennzeichnet, dass** das Reduktionsmittel in Schritt "c" einen pH von6,5 bis 10,5 hat.

6. Verfahren nach einem der vorhergehenden Ansprüche, **dadurch gekennzeichnet, dass** die im Schritt "d" verwendete Zusammensetzung mindestens ein Öl, ausgewählt aus natürlichen Ölen, Fettsäure- Fettalkoholestern und Silikonölen enthält, vorzugsweise enthält sie mindestens ein flüchtiges Silikonöl und mindestens ein nicht flüchtiges Silikonöl,

7. Verfahren nach einem der vorhergehenden Ansprüche, **dadurch gekennzeichnet, dass** das in Schritt "e" verwendete Oxydationsmittel mindestens ein Oxydationsmittel in einer Menge von 1 bis 5 Gew.- %, berechnet auf die Gesamtzusammensetzung enthält und einen pH von 2 bis 6 hat.

8. Verfahren nach einem der vorhergehenden Ansprüche, **dadurch gekennzeichnet, dass** die oxidative, färbende Zusammensetzung von Schritt "f" mindestens einen oxydativen Farbentwickler und optional mindestens eine Kupplerverbindung enthält.

9. Verfahren nach einem der vorhergehenden Ansprüche, **dadurch gekennzeichnet, dass** jede Zusammensetzung die in einem der Schritte in dem Verfahren verwendet wird ein Verdickungsmittel, ausgewählt aus anionischen, nicht ionischen und kationischen Polymeren, enthält.

10. Verfahren nach einem der vorhergehenden Ansprüche, **dadurch gekennzeichnet, dass** jede Zusammensetzung die in einem der Schritte in dem Verfahren verwendet eine Emulsion ist und mindestens einen Fettalkohol mit einer Kettenlänge von 12 bis 22 C-Atomen enthält.

11. Verfahren nach einem der vorhergehenden Ansprüche, **dadurch gekennzeichnet, dass** jede der Zusammensetzung die in einem der Schritte in dem Verfahren verwendet wird ein Tensid, ausgewählt aus anionischen, kationischen, nicht ionischen und amphoteren Tensiden enthält, wobei das kationische Tensid vorzugsweise aus einer Verbindung der Formel
ausgewählt ist, 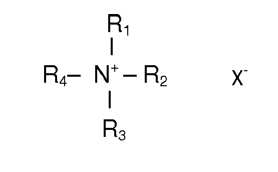 worin R₁ eine gesättigte oder ungesättigte, verzweigte oder nicht verzweigte Alkylkette mit 8 - 22 C- Atomen ist oder
R₅CO NH (CH₂)ₙ
worin R₅ eine gesättigte oder ungesättigte, verzweigte oder nicht verzweigte Alkylkette mit 7 - 21 C- Atomen ist und n einen typischen Werft von 0 - 4 hat oder
R₆CO O (CH₂)ₙ
worin R₆ eine gesättigte oder ungesättigte, verzweigte oder nicht verzweigte Alkylkette mit 7 - 21 C- Atomen ist und n einen typischen Wert von o - 4 hat und
R₂ ein Wasserstoff, eine gesättigte oder ungesättigte, verzweigte oder nicht verzweigte Alkylkette mit 1 - 22 C- Atomen ist oder
R₅CO NH (CH₂)ₙ
worin R₅ eine gesättigte oder ungesättigte, verzweigte oder nicht verzweigte Alkylkette mit 7 - 21 C- Atomen ist und n einen typischen Wert von 0 - 4 hat oder
R₆ CO O (CH₂)ₙ
worin R₆ eine gesättigte oder ungesättigte, verzweigte oder nicht verzweigte Alkylkette mit 7 - 21 C- Atomen ist und n einen typischen Wert von 0 - 4 hat,
und R₃ und R₄ unabhängig voneinander H oder niedrige Alkylketten mit 1 bis 4 Kohlenstoff- Atomen sind und X Chlorid, Bromid oder Methosulfat ist.

12. Verfahren nach einem der vorhergehenden Ansprüche, **dadurch gekennzeichnet, dass** jede der Zusammensetzung die in einem der Schritte in dem Verfahren verwendet wird eine oder mehrere Verbindungen, ausgewählt aus organischen Lösungsmitteln, Silikonverbindungen, Fettsäuren mit 12 bis 22 C- Atomen, kationischen Polymer und Ubichinon der Formel
enthält 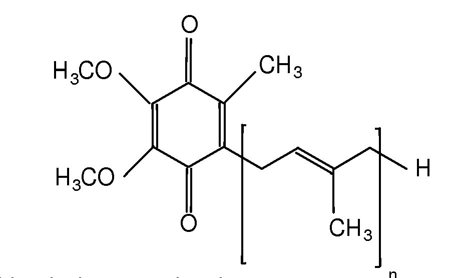 worin n eine Zahl zwischen 1 und 10 ist.

## Revendications

1. Procédé de coloration et de lissage des cheveux en un procédé unique **caractérisé par** les étapes suivantes
a- une composition de coloration aqueuse basée sur au moins un colorant capillaire
direct mais ne comprenant pas de précurseur de colorant d'oxydant ni de substances de couplage, est appliquée sur la chevelure et rincée à l'eau après un temps de pose de 5 à 45 min à une température de 20 à 45°C, shampooing facultatif,
b- séchage avec une serviette,
c- une composition aqueuse comprenant au moins un agent réducteur est appliquée sur
la chevelure et rincée à l'eau après un temps de pose de 5 à 60 min à température ambiante, alors que la chevelure est démêlée continuellement durant le traitement avec un agent réducteur et séchée avec une serviette,
d- facultativement, la chevelure est traitée avec une composition comprenant au moins un sel et est facultativement rincée,
e- une composition comprenant au moins un agent oxydant est appliquée sur la
chevelure traitée pendant 2 à 15 min et est facultativement rincée à l'eau et facultativement séchée avec une serviette, et
f- une composition de coloration aqueuse basée sur au moins un colorant capillaire
direct est, facultativement, mélangée avec une composition comprenant au moins un agent oxydant et appliquée sur la chevelure puis rincée à l'eau après avoir laissé agir 5 à 45 min à une température de 20 à 45°C puis séchée avec une serviette et séchée au sèche-cheveux ou laissée sécher à l'air libre.

2. Le procédé selon la revendication 1, **caractérisé par le fait que** les compositions de coloration ont un pH compris entre 2 et 12 et au moins un colorant capillaire direct est choisi parmi les colorants nitro anioniques, les colorants nitro cationiques et les colorants nitro neutres ou leurs mélanges.

3. Le procédé, selon les revendications 1 et 2, **caractérisé par le fait que** la composition de coloration dans l'étape "a" est mélangée avec une composition comprenant au moins un agent oxydant et a un pH de 5 à 12 après mélange.

4. Le procédé, selon les revendications de 1 à 3, **caractérisé par le fait que** la composition réductrice de l'étape "c" comprend au moins un agent réducteur choisi parmi les suivants :
acide thioglycolique, acide thiolactique et/ou leurs sels, en particulier les sels d'ammonium et d'éthanolamine, cystéine et/ou les sels hydrochlorures en résultant, homocystéine, cystéamine, N-acétylcystéine, thioglycérol, monothioglycolate d'éthylène glycol,
monothioglycolate de 1,2-propylèneglycol, monothioglycolate de 1,3-propanediol ou le mélange isomère en résultant, monothioglycolate de 1,4-butanediol et les mélanges isomères en résultant, polyéthylène glycol tel que les monothioglycolates de di-, tri- et
tetraéthylèneglycol, monothioglycolate de glycérol et d'autres acides thio et les éthers en résultant, ainsi que les mélanges en résultant à une concentration en poids de 2 à 15% calculée sur la composition totale.

5. Le procédé selon l'une des revendications précédentes quelconque, **caractérisé par le fait que** la composition réductrice de l'étape "c" a un pH compris entre 6,5 et 10,5.

6. Le procédé selon l'une des revendications précédentes quelconque, **caractérisé par le fait que** la composition utilisée dans l'étape "d" comprend au moins une huile choisie parmi les huiles naturelles, les acides gras, les esters d'alcool gras et les huiles silicones, de préférence au moins une huile silicone volatile et au moins une huile silicone non-volatile.

7. Le procédé selon l'une des revendications précédentes quelconque, **caractérisé par le fait que** l'agent oxydant utilisé dans l'étape "e" comprend au moins un agent oxydant à une concentration en poids de 1 à 5% calculée sur la composition totale et a un pH de 2 à 6.

8. Le procédé selon l'une des revendications précédentes quelconque, **caractérisé par le fait que** la composition de coloration d'oxydation de l'étape "f" comprend au moins un précurseur de colorant oxydant et, en option, au moins un agent de couplage.

9. Le procédé selon l'une des revendications précédentes quelconque, **caractérisé par le fait que** toutes les compositions utilisées dans toutes les étapes du procédé comprennent un agent épaississant choisi parmi les polymères anioniques, cationiques et non-ioniques.

10. Le procédé selon l'une des revendications précédentes, **caractérisé par le fait que** toutes les compositions utilisées dans toutes les étapes du traitement sont une émulsion et comprennent au moins un alcool gras avec une chaîne alkyle longue de 12 à 22 atomes de carbone.

11. Le procédé selon l'une des revendications précédentes quelconque, **caractérisé par le fait que** toutes les compositions utilisées dans toutes les étapes du traitement comprennent un agent tensioactif choisi parmi les agents tensioactifs anioniques, cationiques, non-ioniques et amphotères, où l'agent tensioactif cationique est de préférence choisi parmi les composés de formule suivante : 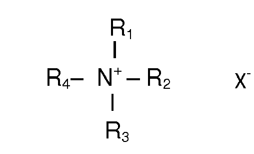 où R₁ est une chaîne alkyle saturée ou insaturée, ramifiée ou non-ramifiée avec 8-22 atomes C ou
R₅CO NH (CH₂)ₙ
où R₅ est une chaîne alkyle saturée ou insaturée, ramifiée ou non-ramifiée avec 7-21 atomes C et n a une valeur typique de 0 - 4 ou
R₆CO O (CH₂)ₙ
où R₆ est une chaîne alkyle saturée ou insaturée, ramifiée ou non-ramifiée avec 7-21 atomes C et n a une valeur typique de 0 - 4 et
R₂ est un atome d'hydrogène, une chaîne alkyle saturée ou insaturée, ramifiée ou non-ramifiée avec 1-22 atomes C ou
R₅CO NH (CH₂)ₙ
où R₅ est une chaîne alkyle saturée ou insaturée, ramifiée ou non-ramifiée avec 7-21 atomes C et n a une valeur typique de 0 - 4 ou
R₆ CO O (CH₂)ₙ
où R6 est une chaîne alkyle saturée ou insaturée, ramifiée ou non-ramifiée avec 7-21 atomes C et n a une valeur typique de 0 - 4,
et R₃ et R₄ sont, indépendamment, un atome d'hydrogène ou une chaîne alkyle inférieure avec 1 - 4 atomes de carbones, et X est un atome de chlorure, bromure ou méthosulfate.

12. Le procédé selon l'une des revendications précédentes quelconque, **caractérisé par le fait que** toutes les compositions utilisées dans toutes les étapes comprennent au moins un plusieurs composés choisis parmi les solvants organiques, les composés siliconés, les acides gras avec 12 à 22 atomes de carbone, les polymères cationiques et les ubiquinones de formule suivante : 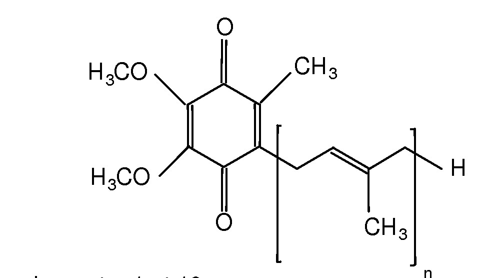 où n est un nombre entre 1 et 10.
